(19) Europäisches Patentamt
European Patent Office
Office européen des brevets

(11) **EP 3 182 109 A1**

(12) **EUROPEAN PATENT APPLICATION**
published in accordance with Art. 153(4) EPC

(43) Date of publication:
**21.06.2017 Bulletin 2017/25**

(51) Int Cl.:
**G01N 27/30** (2006.01)

(21) Application number: **15830450.1**

(22) Date of filing: **03.08.2015**

(86) International application number:
**PCT/CN2015/085929**

(87) International publication number:
**WO 2016/019836 (11.02.2016 Gazette 2016/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **07.08.2014 CN 201410385603**

(71) Applicant: **Ocular Fluidics, Inc.
Moraga, CA 94556 (US)**

(72) Inventors:
• HUANG, Chengjun
  **Beijing 100029 (CN)**
• LUO, Jun
  **Beijing 100029 (CN)**
• ZHAO, Chao
  **3010 Kessel-Lo (BE)**

(74) Representative: **Hally, Anna-Louise
FRKelly
27 Clyde Road
Dublin D04 F838 (IE)**

(54) **SENSING APPARATUS**

(57) A sensing device is disclosed. The sensing device comprises: a first electrode layer, a second electrode layer, which are separated by a dielectric layer; and through holes penetrating through the first electrode layer, the second electrode layer and the dielectric layer.

Fig. 2

**Description**

**Cross-References**

The present disclosure claims priority of Chinese Patent Application No. 201410385603.8 entitled "Sensing Device" filed on August 7, 2014, which is incorporated herein by reference in its entirety.

**Technical Field**

The disclosure generally relates to electrochemical detection, and more specifically, to a sensing device, such as an electrochemical biosensor.

**Background**

An electrochemical biosensor comprises a biological sensitive element and an electrochemical sensor. The biological sensitive element comprises early enzyme catalyst electrode, and later DNA, antigen, antibody, animalcule, and animal and plant tissues. Performance of such sensors are continuously improved by various modern biological techniques and chemical modification techniques.

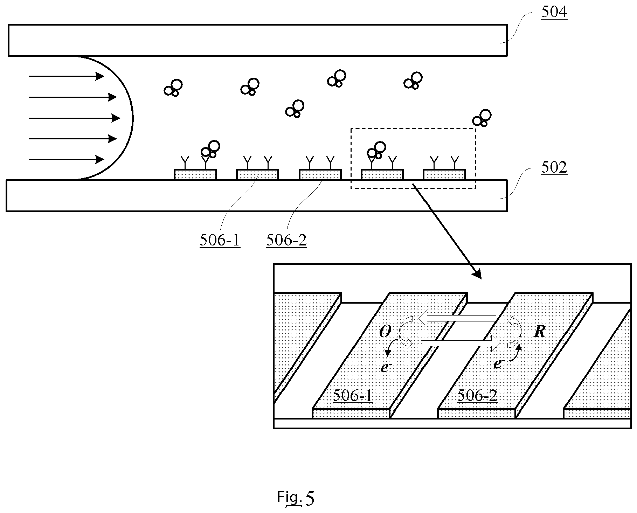

In conventional electrochemical sensors as illustrated in Figure 5, detecting electrodes 506-1, 506-2 are generally fabricated on a silicon substrate or other substrates 502. Probe molecules, indicated by Y-type symbols in the drawing, may be attached to electrodes 506-1, 506-2. When fluid samples flow over the channel defined by the substrate 502 and the cover plate 504, as indicated by the horizontal arrow in the drawing, a biochemical reaction may occur between the molecules to be detected in the samples (as indicated by the circle in the drawing) and the probe molecules. Variation of charges/currents during the biochemical reaction (generally, redox reaction) may be detected by the electrodes 506-1 and 506-2. Typically, the electrode may have a structure of an Interdigitated microelectrodes Array (IDA). In such a case, two adjacent electrodes, which can be used as an oxidation electrode and a reduction electrode, respectively, constitute a group of electrode pair and may be applied with different bias voltages. The process may be cycled and reciprocated, and may be referred as redox cycling. As illustrated in Figure 5, in a redox cycling, the reduction product (R) on the cathode 506-2 arrives at the anode 506-1 via diffusion and is oxidized. Then, the oxidation product (O) diffuses to the cathode and is reduced, and finally the cycling is completed.

In the redox cycling, the path along which the reaction products diffuse and the time for the diffusion depend on the distance between the cathode 506-2 and the anode 506-1. The efficiency of the redox cycling can be improved by decreasing the spacing between adjacent electrodes, so as to increase the electrochemical detecting signals. Quantitatively, the steady-state current $I_{limiting}$ in the redox cycling reaction can be derived by:

$$I_{\text{limiting}} = nFC_{\text{spe}}Dmb\left[0.637\ln\frac{2.55(w+g)}{g} -0.19\left(\frac{g}{w+g}\right)^{2}\right]$$

wherein g is the spacing between the adjacent electrodes, w is the width of the electrode, m is the number of the Interdigitated electrode pairs, b is the length of the electrode, F is the Faraday constant, n is the number of the transferred electrons in the redox reaction, $C_{spe}$ is the concentration of the redox molecules, and D is the diffusion coefficient of the redox molecules. According to the computation for the relationship between the steady-state current and the spacing of the interdigitated electrode pairs in a typical experimental condition, the steady-state current may decrease rapidly when the spacing is larger than several microns.

In order to make full use of the characteristics of the redox cycling and to increase the detecting current as large as possible, electrodes in micron or even nanometer scale are generally employed, and the spacing between electrodes are reduced as small as possible. In conventional methods, the spacing between electrodes in nanometer scale are fabricated by improving micro-nano fabricating process, for example, by deep ultraviolet lithography or electron beam lithography. However, the process difficulty and cost for fabricating nano-scale electrodes are increasing enormously with the scaling of the element. If electron beam lithography is employed, current nano-scale electrodes may have a dimension of about several nanometers, and long exposure time is required, which lead to lower yield.

Furthermore, in such an electrochemical detecting device, the molecules to be detected are transported in a flow-over mode, as illustrated in Figure 5. Specifically, the molecules to be detected have to diffuse in the vicinity of the probe molecules in order to have a binding reaction. In a laminar flow state in micro-scale, binding efficiency of the molecules to be detected and the probe molecules is restricted.

**Summary of Invention**

An object of the present disclosure is to provide, among others, a sensing device, which can efficiently detect targeting particles, such as chemical and/or biological molecules.

According to embodiments of the present disclosure, a sensing device is provided. The sensing device comprises: a first electrode layer, a second electrode layer, which are separated by a dielectric layer; and through holes penetrating through the first electrode layer, the second electrode layer and the dielectric layer.

In the sensing device according to embodiments of the present disclosure, spacings between electrodes in, for example, several to several tens of nanometers, are naturally formed by means of the dielectric layer between two electrode layers (which constitute a three-dimensional electrode configuration) without the complicated and expensive electron beam lithography process.

Furthermore, in conventional electrochemical sensors (as shown in Figure 5), molecules to be detected flow over the device (electrode). In a case where laminar-flow in micro-scale occurs, the reaction of molecules to be detected with probe molecules is mainly controlled by the diffusion speed of the molecules to be detected, which may lead to lower detecting speed and worse sensitivity. According to embodiments of the present disclosure, a novel micro/nano through hole structure is provided. Electrodes are disposed in different levels and fluid samples flow through the through holes, which greatly enhances diffusion efficiency of the molecules to be detected and probability with which the probe molecules react, and improves sensor sensitivity.

**Description of Drawings**

The above and other objects, features, and advantages of the present disclosure will become more apparent from the following descriptions of embodiments thereof with reference to attached drawings, in which:

Figure 1 schematically illustrates a perspective view of a sensing device according to an embodiment of the present disclosure;

Figure 2 schematically illustrates a cross-sectional view of a sensing device according to another embodiment of the present disclosure;

Figure 3 schematically illustrates a cross-sectional view of a sensing device according to a further embodiment of the present disclosure;

Figure 4 schematically illustrates a cross-sectional view of a sensing device according to a still further embodiment of the present disclosure; and

Figure 5 illustrates a schematic view of an electrochemical sensor in related art.

**Detailed Description**

Hereinafter, descriptions are given for embodiments of the present disclosure with reference to the attached drawings. However, it is to be understood that these descriptions are illustrative and not intended to limit the present disclosure. Further, in the following, known structures and technology are not described to avoid obscuring concepts of the present disclosure unnecessarily.

In the drawings, various structures according to embodiments of the present disclosure are schematically shown. However, they are not drawn to scale, and some features may be enlarged while some features may be omitted for sake of clarity. Moreover, shapes and relative sizes and positions of regions and layers shown in the drawings are only illustrative, and deviations may occur due to manufacture tolerances and technique limitations in practice. Those skilled in the art can also devise regions/layers of other different shapes, sizes, and relative positions as desired.

In the context of the present disclosure, when a layer/element is recited as being "on" a further layer/element, the layer/element can be disposed directly on the further layer/element, or otherwise there may be an intervening layer/element interposed therebetween. Further, if a layer/element is "on" a further layer/element in an orientation, then the layer/element can be "under" the further layer/element when the orientation is turned.

According to embodiments of the present disclosure, a sensing device is provided to detect targeting particles in samples, such as chemical and/or biological molecules. The sensing device comprises a first electrode layer, a second electrode layer, which are separated by a dielectric layer, and through holes penetrating through the first electrode layer, the second electrode layer and the dielectric layer. A through hole array may be formed in a cribriform structure.

The sandwich structure of the first and second electrode layers and the dielectric layer can be disposed on one side of a substrate, and the through holes may penetrate through the substrate. Optionally, a further sandwich structure of a further first electrode layer, a further second electrode layer and a further dielectric layer can also be disposed on the opposite side of the substrate. And the through holes may also penetrate through the further sandwich structure.

The through holes may have various types as appreciate (for example, for easy manufacturing), such as a substantially circular type. The through holes in the array may have the same or different types, and the respective through holes may have the same or different dimensions. The through holes may perpendicularly penetrate through the sandwich structure of the first electrode layer, the second electrode layer and the dielectric layer (and optionally, the substrate thereunder). Each through hole may have the same or different dimension in the first and second electrode layers.

According to embodiments of the present disclosure, the sensing device may comprise a microfluidic chip which is configured to introduce fluid samples into the device such that the samples can flow through the through holes.

Such sensing devices can be used as electrochemical biosensors.

The technology of the present disclosure can be implemented in various ways, some of which are exemplified in the following with reference to the drawings.

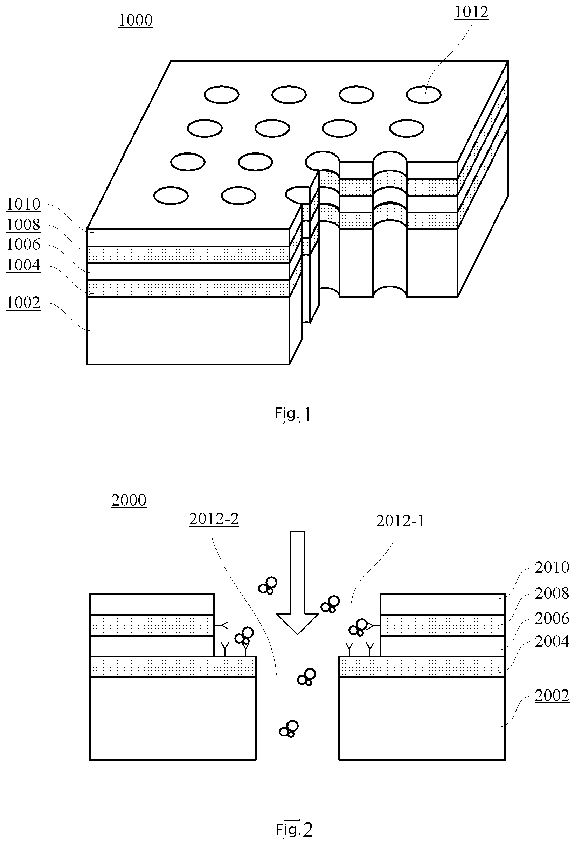

Figure 1 schematically illustrates a perspective view of a sensing device according to an embodiment of the present disclosure.

As shown in Figure 1, the sensing device 1000 according to the embodiment can comprise a substrate 1002. For example, the substrate 1002 may comprise at least one of semiconductor materials, such as silicon, inorganic materials, such as glass and quartz, and polymers, such as polymethyl methacrylate and polycarbonate.

A first electrode layer 1004, a dielectric layer 1006 and a second electrode layer 1008 are sequentially formed on the substrate 1002. Optionally, a passivation layer 1010 may be formed on the second electrode layer 1008 to protect the respective layers thereunder. The respective layers can be formed on the substrate 1002 by, for example, deposition or evaporation. The first electrode layer 1004 and the second electrode layer 1008 may comprise appropriate conducting materials, for example, metals, such as Au, and may have a thickness of about several to hundreds of nanometers. In order to enhance adhesion between the electrode layer and the substrate 1002 or between the electrode layer and the dielectric layer 1006, a transition layer may be formed between the electrode layer and the substrate 1002 and/or between the electrode layer and the dielectric layer 1006. The transition layer may comprise conducting materials as appropriate, for example, metals, such as Ti, Cr, etc., and may have a thickness of about several to several tens of nanometers. The dielectric layer 1006 comprises dielectric materials as appropriate, for example, silicon oxide, silicon nitride, etc., and may have a thickness of about several to several tens of nanometers. The passivation layer 1008 may comprise silicon oxide, silicon nitride or other polymers, and may have a thickness of about several to hundreds of nanometers.

It should be noted that, the substrate beneath the sandwich structure of the electrode layers and the dielectric layer and the passivation layer on the sandwich structure are schematically shown in Figure 1. However, they are optional. In some applications, the substrate and/or the passivation layer may be even omitted.

In the sandwich structure, through holes 1012 can be formed to penetrate through the sandwich structure on opposite sides (in the drawings, upper and lower sides) by, for example, an etching process. For example, the through holes 1012 may have a circular type and may have a diameter of about 100nm-500$\mu$m. In a case where the substrate 1002 and/or the passivation layer 1010 are formed, the through holes 1012 also penetrate through the substrate 1002 and/or the passivation layer 1010. The fluid can flow through the through holes from one side of the device (for example, the upper side in Figure 1) to the other side (for example, the lower side in Figure 1), such that the fluid can flow through the first and second electrode layers, and electrochemical detecting is achieved with high efficiency.

Though an array of 4×4 through holes is shown in Figure 1, the present disclosure is not limited thereto. There may be more or less through holes. Further, the array in Figure 1 is an array having a regular square shape. However, the present disclosure is not limited thereto. The through holes may be disposed in other regular or irregular patterns. The shapes of the through holes are not limited to the regular columniform shown in the drawings. The through holes may have any other shape which is suited for manufacturing, and may comprise variations in shape caused by manufacturing tolerance, process limitation, etc.

Further, in the example in Figure 1, the sandwich structure of the electrode layers and the dielectric layer is only formed on one side (upper side in Figure 1) of the substrate 1002. However, the present disclosure is not limited thereto. For example, another sandwich structure of the electrode layers and the dielectric layer can be formed on the opposite side (lower side in Figure 1) of the substrate 1002. The electrode layers and the dielectric layer in the another sandwich structure may have the same or different configuration as that of the above sandwich structure.

Figure 2 schematically illustrates a cross-sectional view of a sensing device according to another embodiment of the present disclosure.

As shown in Figure 2, the sensing device 2000 according to the embodiment can comprise a substrate 2002. A first electrode layer 2004, a dielectric layer 2006, a second electrode layer 2008 and a passivation layer 2010 are sequentially formed on the substrate 2002. Description of the configuration of the substrate and these layers can be referred to explanation given with reference to Figure 1.

The sensing device 2000 can also comprise a through hole (2012-1 and 2012-2) penetrating through the substrate 2002 and respective layers thereon. In the embodiment, the through hole has a dimension in the second electrode layer 2008 (2012-1) different (in this embodiment, larger) from that in the first electrode layer 2004 (2012-2). For example, such a through hole can be manufactured as follows. Specifically, the passivation layer 2010, the second electrode layer 2008 and the dielectric layer 2006 are sequentially etched by, for example, Reactive Ion Etching (RIE) by means of a first photomask. The first photomask can define an opening with a relative large size. Next, the first electrode layer 2004 and the substrate 2002 are sequentially etched by means of a second photomask. The second photomask can define an opening with a relative small size.

The probe molecules, for example, antibody protein, as indicated by Y-type symbols in the drawings, can be attached to surfaces of the through holes exposed in the first electrode layer 2004 and the second electrode layer 2008. Electrical signals, such as direct or alternating current signals, can be applied to the first electrode layer 2004 and the second electrode layer 2008. Those skilled in the art can conceive various means to form connections such as wirings to apply electrical signals to the first electrode layer 2004 and the second electrode layer 2008. When the fluid samples flow through the through hole along a direction indicated by the arrow in the drawing, the molecules to be detected in the samples (indicated by the circular symbol in the drawing) may react with the probe molecules, so as to achieve detection of the molecules to be detected. For particular molecules to be detected, it is apparent for selection of probe molecules for those skilled in the art.

It should be noted that only a single through hole (2012-1 and 2012-2) is shown in Figure 2 for the sake of convenience. However, the present disclosure is not limited thereto. There may exist more through holes.

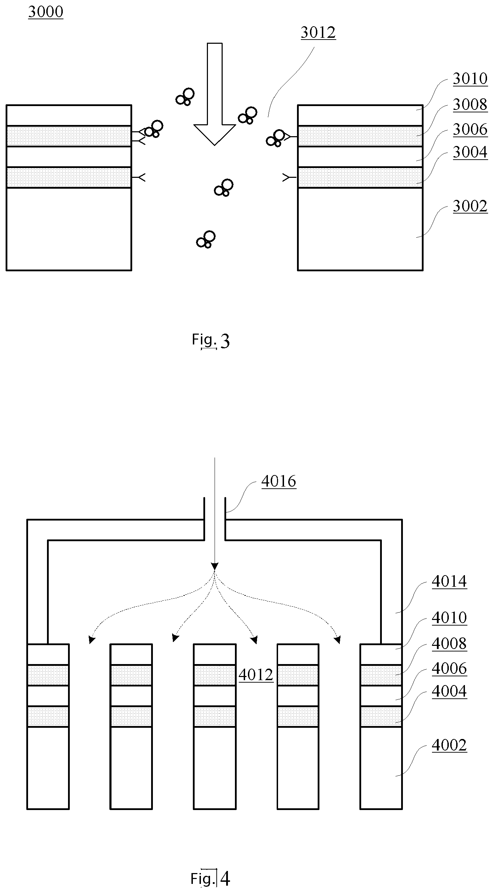

Figure 3 schematically illustrates a cross-sectional view of a sensing device according to a further embodiment of the present disclosure. The sensing device 3000 is substantially the same as the sensing device 2000 in Figure 2 except that the through holes have different shapes.

As shown in Figure 3, the sensing device 3000 according to the embodiment can comprise a substrate 3002. A first electrode layer 3004, a dielectric layer 3006, a second electrode layer 3008 and a passivation layer 3010 are sequentially formed on the substrate 3002. Description of the configuration of the substrate and these layers can be referred to explanation given with reference to Figure 1.

The sensing device 3000 can also comprise a through hole penetrating through the substrate 3002 and respective layers thereon. In the embodiment, the through hole have a dimension in the second electrode layer 3008 substantially the same as that in the first electrode layer 3004. Specially, in the embodiment, the through hole has cross sections in substantially the same size to penetrate through the respective layers. For example, such a through hole can be manufactured as follows. Specifically, the passivation layer 3010, the second electrode layer 3008, the dielectric layer 3006, the first electrode layer 3004 and the substrate 3002 are sequentially etched by, for example, Reactive Ion Etching (RIE) by means of the same photomask.

The probe molecules, for example, antibody protein, as indicated by Y-type symbols in the drawings, can be attached to surfaces of the through hole exposed in the first electrode layer 3004 and the second electrode layer 308. Electrical signals, such as direct or alternating current signals, can be applied to the first electrode layer 3004 and the second electrode layer 3008. When the fluid samples flow through the through hole along a direction indicated by the arrow in the drawing, the molecules to be detected in the samples (indicated by the circular symbol in the drawing) may react with the probe molecules, so as to achieve detection of molecules to be detected.

Figure 4 schematically illustrates a cross-sectional view of a sensing device according to a still further embodiment of the present disclosure.

As shown in Figure 4, the sensing device can comprise a substrate 4002. A first electrode layer 4004, a dielectric layer 4006, a second electrode layer 4008 and a passivation layer 4010 are sequentially formed on the substrate 4002. The sensing device can also comprise through holes penetrating through the substrate 4002 and respective layers thereon. Description of the configuration of the substrate and these layers can be referred to explanation given with reference to Figures 1-3.

The device can also comprise a microfluidic chip 4014. The microfluidic chip 4014 can comprise an inlet 4016 for introducing fluid samples containing molecules to be detected into the device such that the fluid sample can flow through the through holes 4012. Though only one inlet for sample loading is shown in Figure 4, the present disclosure is not limited thereto. The microfluidic chip can comprise more inlets.

The microfluidic chip can precisely control and manipulate fluid in micro-scale. For example, the microfluidic chip may be manufactured of transparent polymers, such as Polymethylmethacrylate (PMMA), Polycarbonate (PC), Polydimethylsiloxane (PDMS), etc., and may have micro-structures, such as micro-channels, micro-cavities, etc., manufactured by microfabrication techniques. The micro-structures have at least one dimension in micro-scale among scales such as length, width, height, etc. A closed channel can be formed by bonding the microfluidic chip with structures thereunder or by applying pressure, so as to transport fluid.

Various features are described in different embodiments in the above descriptions. However, it is not implied that these features cannot be combined advantageously.

In the above, embodiments of the present disclosure are described. However, such embodiments are given for illustrative only, rather than limiting the scope of the present disclosure. The scope of the present disclosure is defined by appended claims and equivalents thereof. Without departing from the scope of the present disclosure, those skilled in the art can make various alternations and modifications which fall within the scope of the present disclosure.

**Claims**

1. A sensing device, comprising:

   a first electrode layer and a second electrode layer, which are separated by a dielectric layer; and
   through holes penetrating through the first electrode layer, the second electrode layer and the dielectric layer.

2. The sensing device of claim 1, further comprising: a substrate, wherein the first electrode layer, the dielectric layer and the second electrode layer are sequentially formed on one side of the substrate, and wherein the through holes penetrate through the substrate.

3. The sensing device of claim 1, wherein the through holes have a dimension in the first electrode layer different from that in the second electrode layer.

4. The sensing device of claim 3, wherein the through holes have a dimension in the dielectric layer substantially the same as that in the second electrode layer.

5. The sensing device of claim 1, wherein the through holes have a dimension in the first electrode layer substantially the same as that in the second electrode layer.

6. The sensing device of claim 5, wherein the through holes have a dimension in the dielectric layer substantially the same as that in the first and second electrode layers.

7. The sensing device of claim 1, wherein an array of multiple through holes is formed.

8. The sensing device of claim 1, wherein the through holes have a circular shape, and have a diameter of about 100nm-500$\mu$m.

9. The sensing device of claim 2, further comprising: a further first electrode, a further dielectric and a further second electrode layer sequentially formed on the other side opposite to the one side of the substrate, wherein the through holes penetrate through the further first electrode, the further dielectric layer and the further second electrode layer.

10. The sensing device of the claim 1, further comprising: a microfluidic chip, which is configured to introduce fluid samples such that the fluid samples flow through the through holes.

1000

1012

1010
1008
1006
1004

1002

Fig. 1

2000

2012-2

2012-1

2010
2008
2006
2004

2002

Fig. 2

3000

3012

3010
3008
3006
3004

3002

Fig. 3

4016

4014

4010
4008
4006
4004

4012

4002

Fig. 4

504

502

506-1    506-2

O

e⁻

R

e⁻

506-1    506-2

Fig. 5

<table>
<tr><td colspan="2" align="center">**INTERNATIONAL SEARCH REPORT**</td><td>International application No.<br><br>**PCT/CN2015/085929**</td></tr>
</table>

**A. CLASSIFICATION OF SUBJECT MATTER**

G01N 27/30 (2006.01) i

According to International Patent Classification (IPC) or to both national classification and IPC

**B. FIELDS SEARCHED**

Minimum documentation searched (classification system followed by classification symbols)

G01N 27, G01N 33

Documentation searched other than minimum documentation to the extent that such documents are included in the fields searched

Electronic data base consulted during the international search (name of data base and, where practicable, search terms used)

CNABS, WPI, EPODOC, ISI, ELSEVIER: sensor, sensing, biosensor, electrode, through hole, dielectric, substrate, microfluidic

**C. DOCUMENTS CONSIDERED TO BE RELEVANT**

| Category* | Citation of document, with indication, where appropriate, of the relevant passages | Relevant to claim No. |
|---|---|---|
| PX | CN 104792845 A (INSTITUTE OF MICROELECTRONICS OF CHINESE ACADEMY OF SCIENCES), 22 July 2015 (22.07.2015), claims 1-10 | 1-10 |
| X | CN 101454667 B (BAYER HEALTHCARE AG), 24 April 2013 (24.04.2013), description, paragraphs 43, 45, 51-54, 58, 68 and 78, and figures 10a-10d and 12 | 1, 5-7 |
| Y | CN 101454667 B (BAYER HEALTHCARE AG), 24 April 2013 (24.04.2013), description, paragraphs 43, 45, 51-54, 58, 68 and 78, and figures 10a-10d and 12 | 8-10 |
| X | CN 101842698 A (ARKRAY, INC.), 22 September 2010 (22.09.2010), description, paragraphs 33-43, and figures 1-2 | 1-2, 5-6 |
| Y | CN 101842698 A (ARKRAY, INC.), 22 September 2010 (22.09.2010), description, paragraphs 33-43, and figures 1-2 | 9 |
| Y | CN 101960300 A (MATSUSHITA ELECTRIC INDUSTRIAL CO., LTD.), 26 January 2011 (26.01.2011), description, paragraphs 208-214, and figure 18 | 8 |
| Y | WO 2014108082 A1 (BEIJING YICHENG BIOELECTRONICS TECHNOLOGY CO., LTD. et al.), 17 July 2014 (17.07.2014), abstract, and description, pages 7-9 | 10 |

☒ Further documents are listed in the continuation of Box C.   ☒ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 September 2015 (28.09.2015) | **13 October 2015 (13.10.2015)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**HU, Xiaojia**<br><br>Telephone No.: (86-10) **62085681** |

Form PCT/ISA/210 (second sheet) (July 2009)

**INTERNATIONAL SEARCH REPORT**

| International application No. |
| --- |
| **PCT/CN2015/085929** |

**C (Continuation).      DOCUMENTS CONSIDERED TO BE RELEVANT**

| Category* | Citation of document, with indication, where appropriate, of the relevant passages | Relevant to claim No. |
| --- | --- | --- |
| A | WO 2013029115 A1 (NEWSOUTH INNOVATIONS PTY LTD.), 07 March 2013 (07.03.2013), the whole document | 1-10 |
| A | US 2013087455 A1 (JOINSOON MEDICAL TECHNOLOGY CO., LTD.), 11 April 2013 (11.04.2013), the whole document | 1-10 |
| A | JP 2012220291 A (NISSHO KK), 12 November 2012 (12.11.2012), the whole document | 1-10 |

Form PCT/ISA/210 (continuation of second sheet) (July 2009)

## INTERNATIONAL SEARCH REPORT
### Information on patent family members

International application No.

**PCT/CN2015/085929**

| Patent Documents referred in the Report | Publication Date | Patent Family | Publication Date |
|---|---|---|---|
| CN 104792845 A | 22 July 2015 | None | |
| CN 101454667 B | 24 April 2013 | CA 2635670 C | 12 July 2011 |
| | | JP 2009521705 A | 04 June 2009 |
| | | CA 2635670 A1 | 05 July 2007 |
| | | CN 101454667 A | 10 June 2009 |
| | | RU 2008130870 A | 10 February 2010 |
| | | NO 20083293 A | 26 September 2008 |
| | | NO 20083293 B | 26 September 2008 |
| | | EP 1969365 A2 | 17 September 2008 |
| | | US 2009184000 A1 | 23 July 2009 |
| | | BR PI0620735 A2 | 22 November 2011 |
| | | JP 5021678 B2 | 12 September 2012 |
| | | WO 2007076017 A2 | 05 July 2007 |
| CN 101842698 A | 22 September 2010 | EP 2214007 A4 | 26 November 2014 |
| | | JP 5081249 B2 | 28 November 2012 |
| | | US 8384402 B2 | 26 February 2013 |
| | | US 2010244862 A1 | 30 September 2010 |
| | | TW 200938837 A | 16 September 2009 |
| | | KR 101460837 B1 | 11 November 2014 |
| | | WO 2009057792 A1 | 07 May 2009 |
| | | EP 2214007 A1 | 04 August 2010 |
| | | KR 20100101590 A | 17 September 2010 |
| | | TW I435076 B | 21 April 2014 |
| CN 101960300 A | 26 January 2011 | US 2010101965 A1 | 29 April 2010 |
| | | CN 101960300 B | 29 May 2013 |
| | | US 7857963 B2 | 28 December 2010 |
| | | WO 2009144869 A1 | 03 December 2009 |
| | | JP 4418030 B2 | 17 February 2010 |
| WO 2014108082 A1 | 17 July 2014 | CN 103913489 B | 10 June 2015 |
| | | CN 103913489 A | 09 July 2014 |
| WO 2013029115 A1 | 07 March 2013 | US 2014174950 A1 | 26 June 2014 |
| | | AU 2012304200 A1 | 02 May 2013 |
| US 2013087455 A1 | 11 April 2013 | None | |
| JP 2012220291 A | 12 November 2012 | None | |

Form PCT/ISA/210 (patent family annex) (July 2009)

**REFERENCES CITED IN THE DESCRIPTION**

**Patent documents cited in the description**

- CN 201410385603